# EUROPÄISCHE PATENTSCHRIFT

(11) **EP 0 849 261 B1**
(45) Veröffentlichungstag und Bekanntmachung des Hinweises auf die Patenterteilung: **04.10.2001**
(21) Anmeldenummer: 97121720.3
(22) Anmeldetag: 10.12.1997
(51) Int. Cl.: C07D 295/14, C07D 213/74, A61K 31/495

(54) **Piperazinophenyl- und Piperazinophenyloxy-carbonsäure-Derivate sowie Verfahren und Zwischenprodukte zu ihrer Herstellung und diese Verbindungen enthaltende Arzeimittel**
Piperazinophenyl- and piperazinophenyloxy-carboxylic acid derivatives as well as processes and intermediates for their preparation and medicaments containing these compounds
Dérivés d'acides pipérazinophényl- et pipérazinophényloxy-carboxyliques ainsi que procédés et produits intermédiaires de leur préparation et médicaments contenant ces composés

(30) Priorität: 19.12.1996 DE 19652919
(43) Veröffentlichungstag der Anmeldung: 24.06.1998
(73) Patentinhaber: Solvay Pharmaceuticals GmbH, 30173 Hannover (DE)
(72) Erfinder: Kehrbach, Wolfgang, Dr., 30173 Hannover (DE); Schön, Uwe, Dr., 31303 Burgdorf (DE); den Hartog, Jack A.J., Dr., 1382 TA Weesp (NL); van Maarseveen, J.H., Dr., 3813 CX Amersfort (NL); Kruse, C.G., Dr., 3816 Bj Amersfort (NL); Antel, Jochen, Dr., 31848 Bad Münder (DE); Reinders, Jan-Hendrik, Dr., 1216 CW Hilversum (NL); Ziegler, Dieter, Dr., 30966 Hemmingen (DE); Bielenberg, Gerhard-Wilhelm, Dr., 31061 Alfeld, Leine (DE)
(74) Vertreter: Lauer, Dieter, Dr.

(56) Entgegenhaltungen:
- EP-A- 0 542 363
- WO-A-93/14077
- WO-A-96/24583
- WO-A-97/02245
- C. D. ELDRED ET AL.: JOURNAL OF MEDICINAL CHEMISTRY, Bd. 37, 1994, Seiten 3882-5, XP000579663
- B. D. JUDKINS ET AL.: SYNTHETIC COMMUNICATIONS, Bd. 26, Nr. 23, 1996, Seiten 4351-67, XP002059866

## Beschreibung

Die vorliegende Erfindung betrifft neue Piperazinophenylpropion- und Piperazinophenoxyessigsäure-Derivate, welche am Piperazinring durch einen einen Amidinorest tragenden Phenyl- oder Pyridylrest substituiert sind, und deren Salze und biolabile Ester sowie diese Verbindungen enthaltende pharmazeutische Zubereitungen und Verfahren und Zwischenprodukte zur Herstellung dieser Verbindungen.

Aus der europäischen Patentanmeldung, Veröffentlichungs-Nr. 0 542 363 und der internationalen Patentanmeldung, Veröffentlichungs-Nr. WO 93/14077 sind Derivate von Piperidincarbonsäuren bekannt, welche die Fibrinogen-abhängige Blutplättchenaggregation hemmen.

Aufgabe der vorliegenden Erfindung ist es, neue pharmazeutische antithrombotische und blutplättchenaggregationshemmende Wirkstoffe zu entwickeln, die ein günstiges Wirkungsprofil aufweisen.

Es wurde nun gefunden, daß die erfindungsgemäßen neuen Piperazinophenylpropion- und Piperazinophenoxyessigsäure-Derivate wertvolle pharmakologische Eigenschaften besitzen, antithrombotische und blutplättchenaggregationshemmende Wirkungen aufgrund von Fibrinogenrezeptor-antagonistischen Eigenschaften zeigen und ein günstiges Wirkungsprofil mit geringer Toxizität und guter Verträglichkeit aufweisen. Aufgrund ihres Wirkungsprofiles eignen sich die erfindungsgemäßen Substanzen als antithrombotische Wirkstoffe zur Behandlung von auf thrombotischen Prozessen beruhenden Krankheitszuständen.

Die Erfindung betrifft daher neue Verbindungen der allgemeinen Formel I 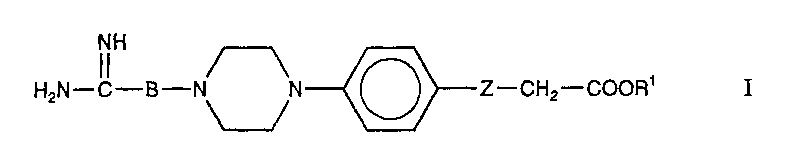 worin
- Z: Sauerstoff oder eine Methylengruppe bedeutet,
- B: einen Phenyl- oder Pyridylrest bedeutet,
- R¹: Wasserstoff oder eine einen biolabilen Ester bildende Gruppe bedeutet
und deren physiologisch verträgliche Säureadditionssalze und physiologisch verträgliche Salze von Säuren der Formel I.

In den Verbindungen der Formel I kann Z für eine Methylengruppe oder für Sauerstoff stehen und ist bevorzugt Sauerstoff.

B kann für einen Phenyl- oder Pyridylrest stehen und ist bevorzugt Phenyl. Die an B gebundene Amidinogruppe ist bevorzugt in para-Stellung zu dem Piperazinring angeordnet.

Die Verbindungen der Formel I stellen gegebenenfalls durch eine einen biolabilen Ester bildende Gruppe veresterte Carbonsäurederivate dar. Als biolabile Ester bildende Gruppen R¹ sind Gruppen geeignet, welche unter physiologischen Bedingungen in vivo unter Freisetzung der Carbonsäuren abgespalten werden können.

Als biolabile Ester bildende Gruppen R¹ eignen sich niedere Alkylgruppen, gegebenenfalls im Phenylring durch niederes Alkyl oder durch eine an zwei benachbarte Kohlenstoffatome gebundene niedere Alkylenkette substituierte Phenyl- oder Phenylniederalkylgruppen oder gegebenenfalls an der Oxymethylgruppe durch niederes Alkyl substituierte C₂-C₆-Alkanoyloxymethylgruppen. Sofern die einen biolabilen Ester bildende Gruppe R¹ niederes Alkyl bedeutet oder enthält, kann dieses verzweigt oder unverzweigt sein und 1 bis 4, vorzugsweise 1 bis 2 Kohlenstoffatome enthalten. Sofern die einen biolabilen Ester bildende Gruppe eine gegebenenfalls substituierte Phenylniederalkylgruppe darstellt, kann deren Alkylenkette 1 bis 3, vorzugsweise 1, Kohlenstoffatome enthalten. Sofern der Phenylring durch eine niedere Alkylenkette substituiert ist, kann diese 3 bis 4, insbesondere 3 Kohlenstoffatome enthalten. Als phenylhaltige Substituenten R¹ eignen sich insbesondere Phenyl, Benzyl oder Indanyl. Sofern R¹ eine gegebenenfalls substituierte Alkanoyloxymethylgruppe darstellt, kann deren Alkanoyloxygruppe 2 bis 6, vorzugsweise 3 bis 5, Kohlenstoffatome enthalten und ist vorzugsweise verzweigt und kann beispielsweise einen Pivaloyloxymethylrest (= tert.-Butylcarbonyloxymethylrest) darstellen.

Erfindungsgemäß werden die neuen Verbindungen der Formel I und deren Salze erhalten, indem man in an sich bekannter Weise Verbindungen der allgemeinen Formel IIa und deren Salze 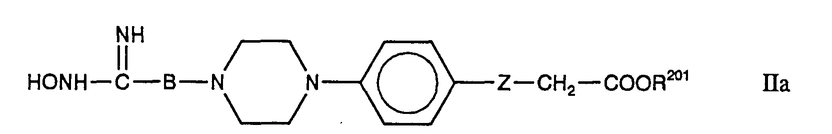 worin Z und B obige Bedeutungen besitzen und R²⁰¹ eine Säureschutzgruppe bedeutet, zu Verbindungen der allgemeinen Formel III 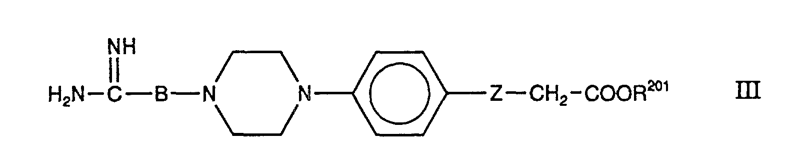 worin Z, B und R²⁰¹ obige Bedeutungen besitzen, hydriert und, sofern die Säureschutzgruppe R²⁰¹ in den Verbindungen der Formel III nicht eine gewünschte einen biolabilen Ester bildende Gruppe darstellt, diese Säureschutzgruppe zur Freisetzung von Säuren der Formel I abspaltet oder die Verbindungen der Formel III oder daraus erhaltene Säuren der Formel I mit einem Alkohol der allgemeinen Formel IVa

**R**^{**101**}**-OH** IVa

worin R¹⁰¹ die für R¹ angegebene Bedeutung mit Ausnahme von Wasserstoff besitzt, oder dessen reaktiven Derivaten, zu Verbindungen der Formel Ia 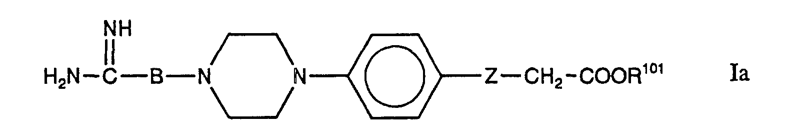 worin Z, B und R¹⁰¹ obige Bedeutungen besitzen, umsetzt, und gewünschtenfalls erhaltene Verbindungen der Formel I in ihre physiologisch verträglichen Säureadditionssalze überführt oder Säuren der Formel I in ihre physiologisch verträglichen Salze überführt oder Säureadditionssalze der Verbindungen der Formel I oder Salze der Säuren der Formel I in die freien Verbindungen überführt.

Als physiologisch verträgliche Säureadditionssalze der Verbindungen der Formel I eignen sich beispielsweise deren Salze mit anorganischen Säuren wie Schwefelsäure, Phosphorsäuren oder Halogenwasserstoffsäuren, insbesondere Chlorwasserstoffsäure oder mit organischen Säuren, beispielsweise niederen aliphatischen Mono-, Di- oder Tricarbonsäuren wie Maleinsäure, Fumarsäure, Milchsäure, Weinsäure, gegebenenfalls durch Halogen substituierte Essigsäure, vorzugsweise Trifluoressigsäure, mit Zitronensäure oder mit Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure.

Freie Säuren der Formel I können als Säuren wie auch als Zwitterionen vorliegen, worin ein im Molekül vorhandenes Stickstoffatom durch das Proton der Carbonsäurefunktion protoniert ist. Im Sinne der vorliegenden Erfindung umfaßt die Formel I daher sowohl biolabile Ester wie freie Säuren und die entsprechenden Zwitterionenformen.

Die Hydrierung der Hydroxyamidine der Formel IIa zu den Amidinen der Formel III kann nach an sich zur katalytischen Hydrierung üblichen Methoden ausgeführt werden. Als Hydrierungskatalysatoren eignen sich beispielsweise Edelmetallkatalysatoren wie Palladium auf Aktivkohle. Die Umsetzung wird in einem unter den Reaktionsbedingungen inerten organischen Lösungsmittel durchgeführt. Als organische Lösungsmittel eignen sich beispielsweise niedere aliphatische Ether wie Tetrahydrofuran oder Di-ethylether, niedere Alkanole wie Methanol oder Ethanol, organische Säuren wie niedere aliphatische Monocarbonsäuren, beispielsweise Essigsäure oder deren Anhydrid, oder Gemische aus den vorgenannten Lösungsmitteln. Ein zur Hydrierung geeigneter Wasserstoffdruck beträgt beispielsweise zwischen 0,5 und 3 bar und liegt vorzugsweise zwischen 1 und 2 bar. Zweckmäßigerweise wird die Hydrierung bei Raumtemperatur durchgeführt.

Als Schutzgruppe R²⁰¹ können an sich zum Schutz von Säurefunktionen übliche Schutzgruppen gewählt werden, die nach an sich bekannten Methoden eingeführt werden und anschließend nach an sich bekannten Methoden wieder abgespalten werden. Geeignete Säureschutzgruppen sind beispielsweise bekannt aus McOmie, "Protective Groups in Organic Chemistry", Plenum Press, und Greene, "Protective Groups in Organic Synthesis", Wiley Intersience Publication. Als Schutzgruppen können auch einen biolabilen Ester bildende Gruppen eingesetzt werden. In diesem Fall stellen die bei der Hydrierung erhaltenen Verbindungen der Formel III bereits erfindungsgemäße Ester der Formel Ia dar.

Aus den durch Hydrierung der Verbindungen der Formel IIa erhaltenen Verbindungen der Formel III kann die Schutzgruppe R²⁰¹, sofern sie keine in den Verbindungen der Formel I gewünschten, einen biolabilen Ester bildende Gruppe darstellt, auf an sich bekannte Weise abgespalten werden.

Zweckmäßig können hydrolytisch abspaltbare Säureschutzgruppen in den Verbindungen der Formel III enthalten sein. Freie Säuren der Formel I können durch an sich bekannte hydrolytische Spaltung dieser Verbindungen der Formel III oder der Ester der Formel Ia erhalten werden. Üblicherweise wird die Hydrolyse in wäßrigem Medium unter sauren Reaktionsbedingungen durchgeführt, wobei Säureadditionssalze von Verbindungen der Formel I gebildet werden können. Zur Durchführung der Hydrolyse geeignete Säuren sind beispielsweise anorganische Säuren, wie Halogenwasserstoffsäuren, insbesondere Salzsäure, sowie organische Säuren wie gegebenenfalls durch Halogen substituierte Essigsäure, beispielsweise Trifluoressigsäure, oder Sulfonsäuren, beispielsweise p-Toluolsulfonsäure oder Methansulfonsäure. Für die saure Esterhydrolyse können gegebenenfalls auch polare aprotische Lösungsmittel wie niedere teilhalogenierte Kohlenwasserstoffe, insbesondere Methylenchlorid verwendet werden. Zur Durchführung der Reaktion sind Temperaturen zwischen Raumtemperatur und der Siedetemperatur des Lösungsmittels geeignet.

Zur Herstellung von Estern der Formel Ia können Säuren der Formel I auf an sich bekannte Weise verestert werden. Für die Veresterung können die freien Säuren der Formel I oder deren Säureadditionssalze oder deren nach allgemein bekannten Verfahren erhaltene reaktionsfähige Derivate wie gemischte Säureanhydride mit Sulfonsäuren oder mit Alkoholen der Formel IVa oder deren reaktiven Derivaten unter zur Esterbildung geeigneten Bedingungen umgesetzt werden. Als geeignet hat sich z. B. die säurekatalysierte Veresterung in einem Überschuß des einzuführenden Alkoholes der Formel IVa erwiesen, der gleichzeitig auch als Lösungsmittel dienen kann. Die Reaktion kann bei Temperaturen, die zwischen Raumtemperatur und der Siedetemperatur des Lösungsmittels liegen, durchgeführt werden. Die zur Katalyse der Veresterungsreaktion eingesetzte Säure kann eine organische Säure wie eine Sulfonsäure oder eine anorganische Säure, beispielsweise eine Halogenwasserstoffsäure sein. Insbesondere kann die Veresterung in 1-normaler alkoholischer Salzsäurelösung durchgeführt werden.

Die Veresterung der freien Säuren der Formel I mit reaktiven Derivaten von Alkoholen der Formel IVa kann auf an sich bekannte Weise stattfinden. Als reaktive Derivate von Alkoholen der Formel IVa eignen sich beispielsweise die aus diesen Alkoholen auf an sich bekannte Weise herstellbaren Halogenide, vorzugsweise deren Chloride oder Bromide, beispielsweise Pivaloyloxymethylchlorid, oder deren Ester mit Niederalkansulfonsäuren wie Methansulfonsäure oder mit gegebenenfalls substituierten aromatischen Sulfonsäuren wie Benzolsulfonsäuren.

Ester der Formel Ia können auch durch Umsetzung von Estern der Formel III mit Alkoholen der Formel IVa nach an sich bekannten Methoden zur Umesterung erhalten werden. Die Umesterung kann zweckmäßig in Lösung stattfinden. Als Lösungsmittel eignet sich ein Überschuß eines zu veresternden Alkoholes der Formel IVa oder ein Gemisch des zu veresternden Alkohols der Formel IVa mit einem unter den Reaktionsbedingungen inerten organischen Lösungsmittel. Als Ausgangsverbindungen für die Umesterung eignen sich alle leicht spaltbaren Ester der Formel III, insbesondere die leicht in saurem Medium spaltbaren tert.-Butylester. Die obigen zur Veresterung der freien Säuren der Formel I geeigneten Reaktionstemperaturen und Lösungsmittel können gleichermaßen auch für die Umesterung verwendet werden. Zur sauren Katalyse der Umesterung können organische Säuren wie Sulfonsäuren oder anorganische Säuren wie Halogenwasserstoffsäuren verwendet werden. Beispielsweise kann die Umesterung in verdünnten alkoholischen Halogenwasserstoffsäuren, insbesondere alkoholischer Salzsäurelösung, beispielsweise in 0,5 bis 3 normaler, bevorzugt 1 bis 2-normaler Salzsäurelösung erfolgen.

Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in physiologisch verträgliche Säureadditionssalze überführt werden.

Verbindungen der allgemeinen Formel IIb 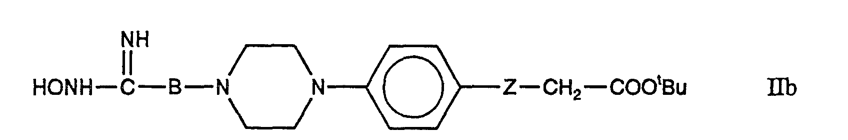 worin B und Z obige Bedeutungen besitzen, können aus den Cyaniden der allgemeinen Formel V 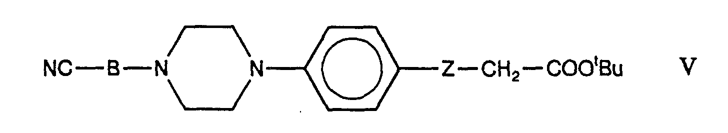 worin B und Z obige Bedeutungen besitzen, durch an sich bekannte Umsetzung mit Hydroxylamin oder einem entsprechenden Salz hiervon erhalten werden. Hierzu kann man eine Cyanidverbindung der Formel V in einem polaren protischen Lösungsmittel, beispielsweise einem niederen Alkanol, bevorzugt Methanol, mit Hydroxylamin oder einem Salz davon, zweckmäßig Hydroxylaminhydrochlorid, in Gegenwart einer organischen Base wie einem niederen Alkalimetallalkoholat, beispielsweise Kalium-tert.-butylat, bei Temperaturen zwischen Raumtemperatur und dem Siedepunkt des Lösungsmittels umsetzen. Um eine möglichst rasche und vollständige Reaktion zu ermöglichen, kann es zweckmäßig sein, unter wasserfreien Bedingungen beim Siedepunkt des Lösungsmittels zu arbeiten und der Reaktionsmischung in gewissen Zeitabständen, beispielsweise alle 4 Stunden, jeweils erneut Hydroxylaminhydrochlorid und Kaliumtert.-butylat bis zur vollständigen Umsetzung zuzusetzen.

Aus den Verbindungen der Formel IIb können Verbindungen der allgemeinen Formel II, 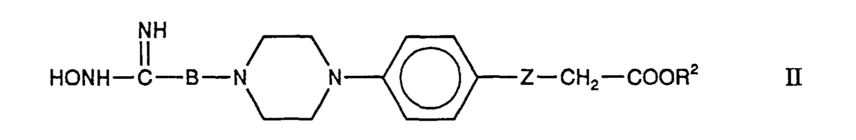 worin B und Z obige Bedeutungen besitzen und R² für Wasserstoff oder eine Säureschutzgruppe R²⁰¹ steht, hergestellt werden, indem man, sofern die tert.-Butylgruppe in den Verbindungen der Formel IIb nicht die gewünschte Säureschutzgruppe darstellt, die tert.-Butylgruppe zur Freisetzung der Säuren der Formel II abspaltet oder die Verbindungen der Formel IIb oder daraus erhaltene Säuren der Formel II mit einem Alkohol der allgemeinen Formel IVb

R²⁰¹-OH IVb

worin R²⁰¹ obige Bedeutung besitzt, oder dessen reaktiven Derivaten, zu Verbindungen der Formel IIa umsetzt. Gewünschtenfalls können erhaltene Verbindungen der Formel II in ihre physiologisch verträglichen Salze überführt werden oder Säureadditionssalze der Verbindungen der Formel II oder Salze der Säuren der Formel II können in die freien Verbindungen überführt werden. Als reaktive Derivate der Alkohole der Formel IVb eignen sich die oben für Alkohole der Formel IVa angegebenen reaktiven Derivate.

Als physiologisch verträgliche Säureadditionssalze der Verbindungen der Formel II eignen sich die oben für Verbindungen der Formel I angegebenen Säureadditionssalze.

Freie Säuren der Formel II können als Säuren wie auch als Zwitterionen vorliegen, worin ein im Molekül vorhandenes Stickstoffatom durch das Proton der Carbonsäurefunktion protoniert ist. Im Sinne der vorliegenden Erfindung umfaßt die Formel II daher sowohl durch Säureschutzgruppen geschützte als auch freie Säuren und die entsprechenden Zwitterionenformen.

Für die Herstellung von Verbindungen der Formel II aus den Verbindungen der Formel IIb eignen sich die oben für die Herstellung der Verbindungen der Formel I aus Verbindungen der Formel III angegebenen Methoden.

Cyanide der Formel V, worin Z für die Methylengruppe steht, können erhalten werden, indem man die Verbindung der Formel VI 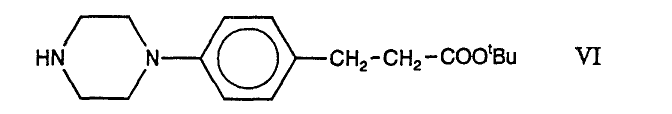 mit den Verbindungen der allgemeinen Formel VII,

**X**^{**1**}**―B―CN** VII

worin B obige Bedeutung besitzt und X¹ eine abspaltbare reaktive Gruppe wie Halogen, insbesondere Chlor oder Fluor, bedeutet, in an sich bekannter Weise umsetzt. Üblicherweise wird die Reaktion in einem organischen, polaren aprotischen Lösungsmittel, beispielsweise Dimethylsulfoxid, Dimethylformamid, Acetonitril oder N-Methyl-2-pyrrolidon bei Temperaturen zwischen Raumtemperatur und 150 °C, bevorzugt zwischen 100° und 140 °C, ausgeführt. Zur Optimierung der Reaktionsbedingungen kann zweckmäßigerweise eine schwache Base, z. B. ein Alkalimetallcarbonat wie Kaliumcarbonat oder Lithiumcarbonat, zugesetzt werden. Ferner kann zweckmäßigerweise nach einer gewissen Reaktionszeit, beispielsweise nach 5 bis 7 Stunden, erneut eine gewisse Menge der Verbindung der Formel VII zugesetzt werden, um den Umsatz zu vervollständigen.

Die Verbindung der Formel VI kann man in an sich bekannter Weise durch Umsetzung der Verbindung der Formel VIII 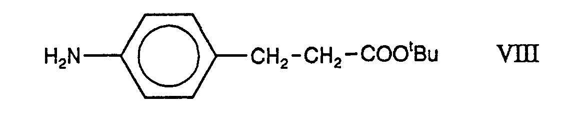 mit Bis-2-Chlorethylamin der Formel IX 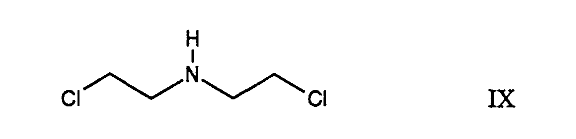 oder einem entsprechenden Salz hiervon, insbesondere dessen Hydrochlorid, erhalten. Zweckmäßig wird die Reaktion in Anwesenheit eines säurebindenden Reagenzes wie einer organischen schwach nukleophilen basischen Stickstoffverbindung, beispielsweise 2,6-Lutidin, Pyridin oder Collidin, in einem unter den Reaktionsbedingungen inerten Lösungsmittel bei Temperaturen zwischen Raumtemperatur und 180 °C, bevorzugt zwischen 120° und 160 °C durchgeführt. Als Lösungsmittel eignen sich aromatische Lösungsmittel wie gegebenenfalls substituierte Benzole, beispielsweise Benzol, Toluol, Xylol oder Chlorbenzol.

Die Verbindung der Formel VIII kann durch an sich bekannte katalytische Hydrierung von 4-Nitrozimtsäure-tert.-butylester der Formel X 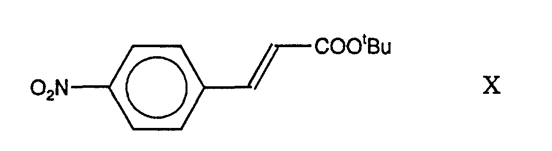 unter den oben für die katalytische Hydrierung der Verbindungen der Formel II zu den Amidinen der Formel III angegebenen Reaktionsbedingungen, bevorzugt in niederen Alkanolen wie Methanol als Lösungsmittel, erhalten werden.

Den Ester der Formel X kann man nach an sich bekannten Methoden der Carbonsäureveresterung durch Umsetzung von 4-Nitrozimtsäurechlorid mit tert.-Butanol herstellen.

Cyanide der Formel V, worin Z Sauerstoff bedeutet, können aus den substituierten Hydroxybenzolen der allgemeinen Formel XI, 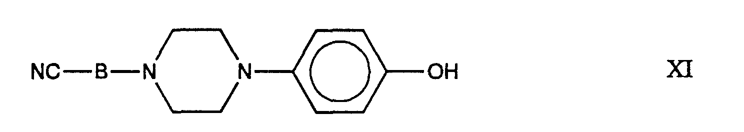 worin B obige Bedeutung besitzt, durch an sich bekannte Umsetzung mit den tert.-Butylestern der allgemeinen Formel XII

X²-CH₂―COO^{t}Bu XII

worin X² eine abspaltbare reaktive Gruppe bedeutet hergestellt werden. X² kann beispielsweise einen Rest einer aliphatischen oder einer gegebenenfalls im Phenylring durch niederes Alkyl oder Halogen substituierten aromatischen Sulfonsäure, wie einen p-Toluolsulfonyloxy-, Phenylsulfonyloxy- oder einen Methansulfonyloxy-Rest, oder ein Halogenatom, insbesondere Brom oder Chlor bedeuten. Bevorzugt stellt X² Chlor dar. Zweckmäßig kann man die Kopplungsreaktion der Verbindungen der Formel XI mit Verbindungen der Formel XII unter den oben für die Umsetzung der Verbindungen der Formel VI mit Verbindungen der Formel VII angegebenen Bedingungen, bevorzugt bei Raumtemperatur, durchführen.

Die Verbindungen der Formel XI können auf an sich bekannte Weise hergestellt werden, indem man Verbindungen der allgemeinen Formel XIII 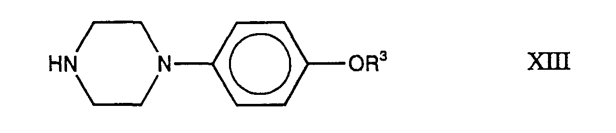 worin R³ für Wasserstoff oder eine geeignete Schutzgruppe für die Alkoholfunktion steht, mit den Verbindungen der Formel VII umsetzt und eine allfällige Schutzgruppe R³ anschließend abspaltet. Die Kopplungsreaktion der Verbindungen der Formel XIII mit Verbindungen der Formel VII kann unter den oben für die Umsetzung der Verbindungen der Formel VI mit Verbindungen der Formel VII beschriebenen Bedingungen, bevorzugt bei Temperaturen von 60° bis 90 °C, ausgeführt werden. Sofern in den Verbindungen der Formel XIII R³ für Wasserstoff steht, ist die Verwendung von Lithiumcarbonat als schwache Base bevorzugt.

Sofern R³ in Verbindungen der Formel XIII für eine Alkohol-Schutzgruppe steht, eignen sich solche Schutzgruppen, die bevorzugt unter reduktiven Bedingungen wieder abgespalten werden können. Geeignete Schutzgruppen sind beispielsweise bekannt aus McOmie, "Protective Groups in Organic Chemistry", Plenum Press. Insbesondere kann R³ eine niedere Alkenylgruppe wie die 2-Propenylgruppe bedeuten. Diese kann unter zur reduktiven Spaltung von Allylethern an sich bekannten Methoden abgespalten werden. Als Reduktionsmittel kann z. B. ein Gemisch aus Ameisensäure und Triethylamin dienen. Die reduktive Spaltung des Allylethers kann beispielsweise in einem polaren Lösungsmittel wie einem Gemisch aus Acetonitril und Wasser und in Anwesenheit eines Katalysators wie einem Palladium-II-Salz, beispielsweise Palladiumacetat, und Triphenylphosphin erfolgen. Die Reaktion kann zwischen Raumtemperatur und dem Siedepunkt des Reaktionsgemisches ausgeführt werden.

Bei der Verbindung der Formel XIII, worin R³ Wasserstoff bedeutet, handelt es sich um an sich bekanntes 1-(4-Hydroxyphenyl)-piperazin.

Verbindungen der allgemeinen Formel XIIIa, 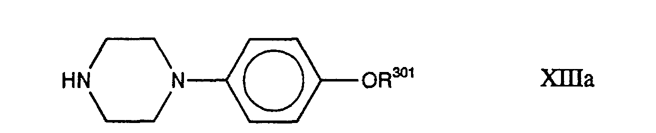 worin R³⁰¹ die Bedeutung von R³ mit Ausnahme von Wasserstoff besitzt, können durch an sich bekannte Reaktion der Amine der allgemeinen Formel XIV, 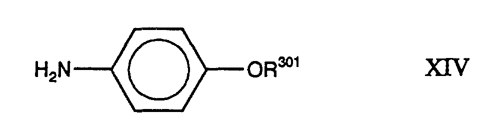 worin R³⁰¹ obige Bedeutung besitzt, mit Bis-2-Chlorethylamin der Formel IX unter den oben für die Umsetzung der Verbindungen der Formel VIII mit Verbindungen der Formel IX angegebenen Bedingungen durchgeführt werden.

Die Amine der allgemeinen Formel XIV können durch an sich bekannte saure Hydrolyse aus den Acetamiden der allgemeinen Formel XV, 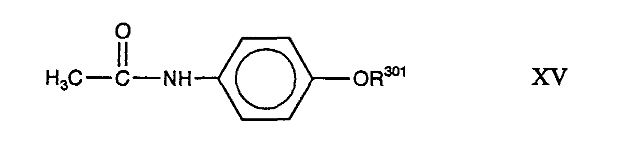 worin R³⁰¹ obige Bedeutung besitzt, freigesetzt werden, beispielsweise durch mehrstündiges Kochen in saurem alkoholischen Medium, wie einem Gemisch aus konzentrierter Salzsäure und Ethanol.

Die Verbindungen der Formel XV können durch Umsetzung von 4-Acetamidophenol der Formel XVI 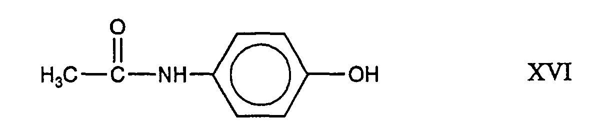 mit einem zur Bildung einer Alkoholschutzgruppe geeigneten Reagens auf an sich bekannte Weise hergestellt werden. Insbesondere kann die Verbindung der Formel XVI nach hierfür bekannten Methoden mit Allylbromid in einen Allylether überführt werden.

Die Verbindungen der Formel I und ihre pharmakologisch akzeptablen Salze zeichnen sich durch interessante pharmakologische Eigenschaften aus. Insbesondere üben die Substanzen eine inhibitorische Wirkung auf die Bindung von Fibrinogen an den Fibrinogenrezeptor der Blutplättchen, das Glykoprotein IIb/IIIa (abgekürzt als GP IIb/IIIa) aus, und eignen sich somit als blutplättchenaggregationshemmende und antithrombotische Wirkstoffe zur Prophylaxe und Behandlung von auf Blutplättchenaggregationsprozessen beruhenden cardiovasculären Beschwerden wie z. B. Thrombosen, Arteriosklerosen und cardialen oder cerebralen Infarkten. Die Blutplättchenaggregation spielt eine wesentliche Rolle bei der Blutgerinnung und Bildung von Blutplättchenpropfen und tritt unter anderem auch bei thrombotischen Prozessen auf, welche zu Aderverstopfungen und cardiovasculären Infarkten führen können. Ein wesentlicher Schritt bei der Bildung von Blutplättchenpfropfen ist das Binden von Fibrinogen an das Blutplättchenmembraneiweiß GP IIb/IIIa, wobei durch das bivalente Fibrinogen Blutplättchen miteinander vernetzt werden. Die erfindungsgemäßen Substanzen entfalten aufgrund ihrer Fibrinogenrezeptor-antagonistischen Eigenschaften ausgeprägte antithrombotische Wirkungen und zeichnen sich durch ein günstiges Wirkungsprofil und gute orale Wirksamkeit aus.

Die Hemmwirkung der Substanzen der Formel I auf die Fibrinogenbindung an den Fibrinogenrezeptor der Blutplättchen, das GP IIb/IIIa, wurde in einem Festphasen-Enzym-Immunoassay vom Typ ELISA ("Enzyme-Linked Immunosorbent Assay") nach der von Kouns et al. (Blood 80 (1992) 2539 - 2547) beschriebenen Methode nachgewiesen.

### Beschreibung der pharmakologischen Versuchsmethode

Eine Lösung von gereinigtem GP IIb/IIIa-Fibrinogenrezeptor aus menschlichen Blutplättchen (1 µg/ml in phosphatgepufferter physiologischer Kochsalzlösung, pH 7,4) wird in Portionen von 100 µl/Probenplatz auf eine Mikrotiterplatte aufgebracht und 18 Stunden lang bei 4 °C stehengelassen. Überschüssiges Rezeptorreagens wird durch eine Standardwäsche (3 mal 350 µl Waschpufferlösung/Probenplatz: 0,01 % (v/v) Tween-80 in phosphatgepufferter physiologischer Kochsalzlösung) abgewaschen und unbesetzt gebliebene Proteinbindungsstellen werden mit einem Blockierungsreagens [300 µl/Probenplatz einer wäßrigen Rezeptorpufferlösung: 20 mMol/l Tris(hydroxy-methyl)-aminomethan, 150 mMol/l NaCl, 1 mMol/l MgCl₂, 1 mMol/l CaCl₂; versetzt mit 5 % (w/v) Rinderserumalbumin (Bovine Serum Albumin, BSA)] besetzt. Man inkubiert 2 Stunden lang bei 37 °C und entfernt überschüssiges Blockierungsreagens.

Anschließend wird gereinigtes menschliches Plasmafibrinogen [100 µl/Probenplatz einer 20 µg/ml Lösung in Testpuffer. Testpuffer = Rezeptorpuffer versetzt mit 1 % (w/v) BSA] zusammen mit der darin gelösten Testsubstanz (Konzentration abhängig von der Testsubstanz) zugegeben und 2 Stunden lang bei 37 °C inkubiert. Man entfernt nichtgebundenes Fibrinogen durch Standardwäsche und setzt die Inkubierung nach Zugabe von biotinylierten Kaninchen-Antikörpern gegen menschliches Fibrinogen (100 µl/Probenplatz in optimaler Gebrauchsverdünnung, gelöst in Testpuffer) eine weitere Stunde bei Raumtemperatur fort. Nach einer weiteren Standardwäsche gibt man Meerrettich-Peroxidase-konjugiertes Streptavidin (1:2000, in geeigneter Verdünnung in Testpuffer) zu, inkubiert wieder eine Stunde bei Raumtemperatur, gefolgt von einer weiteren Standardwäsche.

Schließlich wird das Peroxidase-Substrat 2,2'-Azino-di-(3-ethylbenzthiazolinsulfonat) (im folgenden als ABTS abgekürzt), (100 µl/Probenplatz einer Lösung von 0,4 g/l ABTS in 0,1 mol/l Zitronensäurepuffer, mit 0,4 mol/l NaH₂PO₄·2 H₂O auf pH 4 eingestellt), versetzt mit 0,003 % (w/v) Wasserstoffperoxid, zugegeben und während 20 Minuten die Extinktion des Gemisches bei 405 nm in 5-Minuten-Intervallen gemessen.

Die Peroxidase-Aktivität bestimmt man aus den Meßwerten als Zunahme der Extinktion mit der Zeit. Sie dient zur Berechnung der totalen Fibrinogenbindung sowie der unspezifischen Fibrinogenbindung. Die unspezifische Fibrinogenbindung wird ohne Zusatz des GP IIb/IIIa bestimmt. Aus diesen beiden Werten kann die spezifische Fibrinogenbindung errechnet werden. Der Einfluß der Testsubstanzen wird in IC₅₀-Werten ausgedrückt, die man durch Interpolation aus konzentrationsabhängigen Meßkurven erhält. Die IC₅₀ ist diejenige Konzentration in µMol/l, in welcher eine Testsubstanz die spezifische Fibrinogenbindung an GP IIb/IIIa um 50 % hemmt. Die nachstehende Tabelle 1 gibt die für die Testsubstanz gefundenen IC₅₀ Werte als negative dekadische Logarithmen, den pIC₅₀-Werten, wieder. Die Beispielsnummern beziehen sich auf die nachstehenden Herstellungsbeispiele.

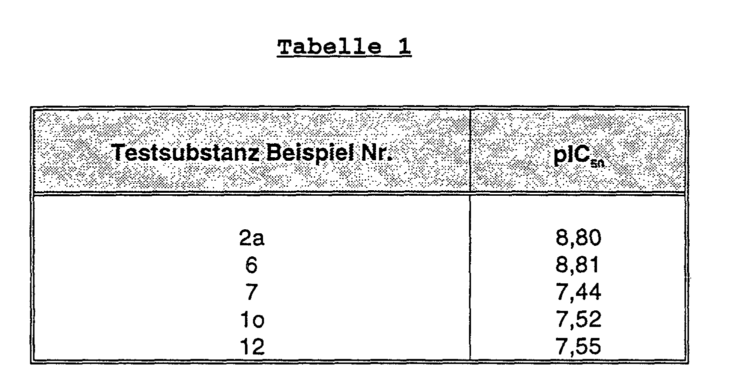

Die vorstehend beschriebenen Testergebnisse zeigen, daß die Verbindungen der Formel I eine ausgeprägte Hemmwirkung auf die Bindung von Fibrinogen an den Fibrinogenrezeptor der Blutplättchen, das GP IIb/IIIa, aufweisen und daher geeignet sind, auf Blutplättchen-Aggregationsprozessen beruhende pathologische Zustände, insbesondere thrombotische Prozesse, günstig zu beeinflussen.

Die vorteilhaften pharmakologischen Eigenschaften der erfindungsgemäßen Verbindungen lassen sich auch in Standard-Tierversuchen nachweisen, beispielsweise durch die Hemmung der ADP-induzierten Thromben-Bildung in der Mikrozirkulation von Hamsterbacken.

Aufgrund ihrer vorstehend beschriebenen Wirkung sind die Verbindungen der Formel I geeignet als Arzneimittel für größere Säugetiere, insbesondere Menschen, zur Behandlung von cardiovasculären Beschwerden wie z. B. Thrombosen, Arteriosklerosen und cardialen oder cerebralen Infarkten. Die zu verwendenden Dosen können individuell verschieden sein und variieren naturgemäß je nach Art des zu behandelnden Zustandes, der verwendeten Substanz und der Applikationsform. Im allgemeinen eignen sich jedoch für Applikationen an größeren Säugetieren, insbesondere Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen, wie z. B. Tabletten, Kapseln, Suppositorien oder Lösungen, enthalten sein. Diese galenischen Zubereitungen können nach an sich bekannten Methoden hergestellt werden unter Verwendung üblicher fester oder flüssiger Trägerstoffe, wie z. B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Die Strukturen der neuen Verbindungen wurden durch spektroskopische Untersuchungen, insbesondere durch Analyse der NMR-, Massen- oder IR-Spektren gesichert.

### Beispiel 1:

### 4-[4-(4-Amidinophenyl)-1-piperazinyl]-phenoxyessigsäuretert.-butylester

A) Unter Stickstoffatmosphäre wurden 15,1 g 1-(4-Hydroxyphenyl)-piperazin, 9,4 g Lithiumcarbonat und 10,4 g 4-Fluorbenzonitril in 250 ml Dimethylsulfoxid bei 80 °C für 4 Stunden zur Reaktion gebracht. Nach erneuter Zugabe von 3,0 g 4-Fluorbenzonitril wurde weitere 4 Stunden bei 80 °C erwärmt und das ausgefallene Produkt nach Abkühlen des Reaktionsgemisches abgesaugt. Zur Abtrennung des mitausgefallenen Lithiumcarbonats wurde das Rohprodukt anschließend mit wäßriger Zitronensäure gerührt. Nach Absaugen und Trocknen im Vakuumschrank bei 60 °C wurden 18,7 g 4-[4-(4-Cyanophenyl)-1-piperazinyl]-phenol erhalten, Fp. = 258 - 262 °C.
B) Zu einer Lösung von 16,0 g des oben erhaltenen Phenols in 150 ml Dimethylformamid wurden 17,2 g Chloressigsäuretert.-butylester, 15,9 g Kaliumcarbonat und 160 mg Kaliumjodid bei Raumtemperatur zugegeben. Nach 13 Stunden wurden weitere 7,0 g Kaliumcarbonat und 8,0 g Chloressigsäuretert.-butylester zugegeben und weitere 2 Stunden gerührt. Nach Zugabe von 1.500 ml Essigsäureethylester wurde 3 mal mit je 300 ml Wasser gewaschen, dann die organische Phase abgetrennt und über Magnesiumsulfat getrocknet. Nach Abfiltrieren des Trockenmittels wurde auf 200 ml Gesamtvolumen eingeengt, das daraufhin auskristallisierte Produkt abfiltriert, mit Diethylether nachgewaschen und über Kaliumhydroxid im Vakuum getrocknet. Man erhielt 18,5 g 4-[4-(4-Cyanophenyl)-1-piperazinyl]-phenoxyessigsäure-tert.-butylester.
C) Eine Lösung von 17,5 g des oben erhaltenen tert.-Butylesters in 1.750 ml Methanol wurde unter Stickstoffatmosphäre am Rückfluß erwärmt. Nach 10 Minuten gab man 1,55 g Hydroxylaminhydrochlorid und 2,5 g Kalium-tert.-butylat zu, während die Lösung weiter unter Rückflußkühlung gekocht wurde. In Abständen von jeweils 4 Stunden wurden 6 weitere Portionen Hydroxylaminhydrochlorid und Kaliumtert.-butylat zugesetzt. Nach vollständiger Umsetzung ließ man auf Raumtemperatur abkühlen, verdünnte mit 300 ml Methylenchlorid und wusch mit gesättigter Kaliumcarbonatlösung. Schließlich wurde die organische Phase abgetrennt, über Magnesiumsulfat getrocknet und eingeengt. Die so erhaltenen weißen Kristalle des 4-[4-(4-Hydroxyamidinophenyl)-1-piperazinyl]-phenoxyessigsäure-tert.-butylesters wurden bei 60 °C getrocknet. Die Ausbeute betrug 11,3 g, Fp. >240 °C (Zersetzung).
D) 10,5 g des vorstehend erhaltenen Hydroxyamidins wurden in 200 ml einer Mischung aus Eisessig und Essigsäureanhydrid (99:1) gelöst und der Ansatz mit Stickstoff gespült. Anschließend wurden 2,0 g 10 %-Palladiumkatalysator auf Aktivkohle hinzugegeben und 6 Stunden lang bei Raumtemperatur und 1 bar Wasserstoffdruck hydriert. Nach Abfiltrieren des Katalysators wurde das Lösungsmittel mit Hilfe von Toluol azeotrop abdestilliert und der verbleibende Rückstand aus Methanol umkristallisiert. Man erhielt 10,0 g eines Acetates der Titelverbindung mit einem Gehalt von ca. 0,7 Mol Essigsäure. Fp. >270 °C (Zersetzung).

### Beispiel 2 :

### 4-[4-(4-Amidinophenyl)-1-piperazinyl]-phenoxyessigsäure.

2a) 5,0 g 4-[4-(4-Amidinophenyl)-1-piperazinyl]-phenoxyessigsäure-tert.-butylester 0,7 Acetat (Herstellung siehe Beispiel 1) wurden in 120 ml 6-normaler wäßriger Salzsäure gelöst und für 1 Stunde bei Raumtemperatur gerührt. Das entstandene Produkt wurde abgesaugt, mit wenig Wasser gewaschen und bei 50 °C im Vakuumtrockenschrank getrocknet. Man erhielt 3,7 g des Dihydrochlorids der Titelverbindung, Fp. >340 °C.
2b) 12,0 g 4-[4-(4-Amidinophenyl)-1-piperazinyl]phenoxyessigsäure-tert.-butylester · 0,7 Acetat wurden in 200 ml eines Gemisches aus Methylenchlorid und Trifluoressigsäure (2:1) gelöst. Nach 5-stündigem Rühren bei Raumtemperatur wurden die leichtflüchtigen Komponenten im Vakuum abgezogen, der Rückstand 3 mal mit jeweils 25 ml Toluol versetzt und jeweils erneut eingeengt. Den so erhaltenen Rückstand nahm man mit einem Gemisch aus Methanol und Methylenchlorid (1:4) auf und fällte das Produkt durch Zugabe von Hexan aus. Nach Filtration und Trocknung erhielt man 12,0 g eines braunen Pulvers, das unter Erwärmen in einem Gemisch aus Methanol und Methylenchlorid gelöst wurde. Nach Abkühlen auf Raumtemperatur wurde das Produkt erneut mit Hexan ausgefällt. Man erhielt 7,25 g des Bistrifluoracetates der Titelverbindung, welches mittels präparativer Hochleistungsflüssigkeitschromatographie (HPLC) weiter gereinigt wurde, Fp. >340 °C.

### Beispiel 3:

### 4-[4-(4-Amidinophenyl)-1-piperazinyl]-phenoxyessigsäuremethylester.

3a) Eine Lösung von 1,0 g 4-[4-(4-Amidinophenyl)-1-piperazinyl]-phenoxyessigsäure-tert.-butylester · 0,7 Acetat in 60 ml 1-normaler methanolischer Salzsäure wurde 40 Stunden lang bei Raumtemperatur gerührt. Die Kristallisation wurde durch Stehenlassen über Nacht im Kühlschrank vervollständigt. Das ausgefallene Produkt wurde abgesaugt, gewaschen und getrocknet. Man erhielt 900 mg des Dihydrochlorids der Titelverbindung, Fp. >330 °C.
3b) 100 mg des Dihydrochlorids von 4-[4-(4-Amidinophenyl)-1-piperazinyl-phenoxyessigsäure (Herstellung siehe Beispiel 2a) wurden in 15 ml 1-normaler methanolischer Salzsäure angeschlämmt und ca. 3 Stunden bei Raumtemperatur gerührt, wobei sich mit zunehmender Reaktionszeit eine klare Lösung bildete. Die Reaktionsmischung wurde eingeengt und im Exsikkator über Kaliumhydroxid getrocknet. Man erhielt 100 mg des Trishydrochlorids der Titelverbindung, Fp. >330 °C.

### Beispiel 4:

### 3-{4-[4-(4-Amidinophenyl)-1-piperazinyl]-phenyl}-propionsäure-tert.-butylester

A) Eine Lösung von 17,8 g 4-Nitrozimtsäurechlorid in 200 ml Tetrahydrofuran wurde zu einer Vorlage aus einer katalytischen Menge 4-Pyrrolidinopyridin und 6,7 ml Pyridin in 900 ml tert.Butanol zugetropft. Nach beendeter Zugabe wurde mit weiteren 200 ml Tetrahydrofuran verdünnt und weitere 5 Stunden bei Raumtemperatur gerührt. Nach dem Abfiltrieren des ausgefallenen Niederschlages wurde das Filtrat mit Methyl-tert.-butylether auf 2 l verdünnt und erst 2 mal mit gesättigter Natriumhydrogencarbonatlösung, dann 2 mal mit gesättigter Kochsalzlösung gewaschen. Trocknen und einengen der organischen Phase lieferte 18,0 g kristallinen 4-Nitrozimtsäure-tert.-butylester.
B) 20,0 g des wie oben erhaltenen 4-Nitrozimtsäureesters wurden in 500 ml Methanol aufgeschlämmt, mit 2,0 g 10%igem Palladium auf Aktivkohle versetzt und 15 Stunden lang bei Raumtemperatur und einem Druck von 4 bar hydriert. Nach dem Abfiltrieren des Katalysators engte man ein und erhielt 15,0 g 4-Aminophenylpropionsäure-tert.-butylester, der ohne weitere Reinigung und Charakterisierung für die folgende Reaktion eingesetzt wurde.
C) Eine Lösung von 11,0 g des vorstehend erhaltenen Aminophenylpropionsäureesters, 10,0 g Bis-(2-chlorethyl)-amin-hydrochlorid und 13 ml Collidin in 150 ml Chlorbenzol wurden 6 Stunden lang bei 140 °C gerührt. Nach dem Abkühlen wurden die ausgefallenen Kristalle abfiltriert und die Mutterlauge eingeengt. Chromatographie an Kieselgel lieferte 6,7 g 3-[4-(1-Piperazinyl)-phenyl]-propionsäure-tert.- butylester.
D) In 100 ml N-Methylpyrrolidon wurden 4,3 g des vorstehend erhaltenen Piperazinylphenylpropionsäureesters, 1,8 g Fluorbenzonitril und 3,0 g Kaliumcarbonat gelöst und 7 Stunden lang bei 120 °C gerührt. Nach Zugabe von weiteren 0,4 g 4-Fluorbenzonitril wurde nochmals 7 Stunden lang auf 120 °C erhitzt. Die ausgefallenen Salze wurden abfiltriert und die Mutterlauge eingedampft. Der Rückstand wurde in Methylenchlorid gelöst und mit Wasser gewaschen. Einengen und Kristallisation aus Ethanol lieferten 3,0 g 3-{4-[4-(4-Cyanophenyl)-1-piperazinyl]-phenyl}-propionsäure-tert.-butylester.
E) 3,0 g des unter D) erhaltenen Phenylpropionsäureesters wurden mit 4 mal 0,25 g Hydroxylaminhydrochlorid und 4 mal 0,38 g Kalium-tert.-butylat nach der in Beispiel 1C) beschriebenen Methode umgesetzt. Man erhielt 1,20 g 3-{4-[4-(4-Hydroxyamidinophenyl)-1-piperazinyl]-phenyl}-propionsäure-tert.-butylester nach Umkristallisation aus Methanol/Methylenchlorid.
F) 0,85 g des vorstehend unter E) erhaltenen Hydroxyamidins und 0,30 g 10 % Palladiumkatalysator auf Aktivkohle wurden nach der in Beispiel 1D) beschriebenen Methode umgesetzt. Man erhielt 0,35 g des Acetats der Titelverbindung. IR: 2977, 1725, 1608 cm⁻¹

### Beispiel 5 :

### 4-{4-[2-(5-Amidinopyridyl)]-1-piperazinyl}-phenoxyessigsäuretert.-butylester

A) 1,0 g 1-(4-Hydroxyphenyl)-piperazin, 650 mg Lithiumcarbonat und 780 mg 2-Chlorpyridin-5-carbonitril wurden in 30 ml Dimethylsulfoxid gelöst und unter Stickstoffatmosphäre 3 Stunden lang bei 80 °C zur Reaktion gebracht. Nach dem Abkühlen wurde der Ansatz eingeengt und das erhaltene Öl in 100 ml 2-molarer Zitronensäurelösung 30 Minuten lang gerührt, wobei das Produkt auskristallisierte. Nach Filtration wurde mit Wasser gewaschen und anschließend getrocknet. Extraktion des Filtrats mit 100 ml Essigsäureethylester lieferte weiteres Produkt, das mit der Hauptmenge vereinigt wurde. Die Ausbeute betrug 1,5 g 4-{4-[2-(5-Cyanopyridyl)]-1-piperazinyl}-phenol.
B) 3,5 g des vorstehend erhaltenen substituierten Phenols wurden zusammen mit 3,6 ml Chloressigsäure-tert.-butylester, 3,45 g Kaliumcarbonat und 25 mg Kaliumjodid in 40 ml Dimethylformamid gelöst und unter Stickstoffatmosphäre 9 Stunden lang bei Raumtemperatur gerührt. Nach erfolgter Reaktion wurde mit Essigsäureethylester verdünnt und 3 mal mit Wasser gewaschen. Die vereinigten organischen Phasen wurden über Natriumsulfat getrocknet und eingeengt. Man erhielt 4,6 g 4-{4-[2-(5-Cyanopyridyl)]-1-piperazinyl}-phenoxyessigsäure-tert.-butylester.
C) Eine Lösung von 4,5 g des vorstehend erhaltenen Phenoxyessigsäureesters in 200 ml absolutem Methanol wurde unter Stickstoffatmosphäre und Rückflußkühlung zum Sieden erhitzt. Nach 10 Minuten gab man 400 mg Hydroxylaminhydrochlorid und 640 mg Kalium-tert.-butylat zu, anschließend in Abständen von 4 Stunden weitere Portionen Hydroxylaminhydrochlorid und Kalium-tert.-butylat, bis zur vollständigen Umsetzung. Nach Abkühlung auf Raumtemperatur wurde mit Methylenchlorid verdünnt und mit 5%iger Kaliumcarbonatlösung gewaschen. Die organische Phase wurde getrocknet und im Vakuum eingeengt. Zur Reinigung wurde aus einer Mischung von Essigsäureethylester und Methanol (1:1) umkristallisiert. Man erhielt 3,6 g 4-{4-[2-(5-Hydroxyamidinophenyl)]-1-piperazinyl}-phenoxyessigsäure-tert.-butylester.
D) In 100 ml eines Gemisches aus Eisessig und Essigsäureanhydrid (99:1) wurden 3,1 g des vorstehend erhaltenen Produktes gelöst. Der Ansatz wurde mit Stickstoff gespült, anschließend wurde 1,0 g 10%iger Palladiumkatalysator auf Aktivkohle zugegeben und 4 Stunden lang bei einem Druck von 1 bar hydriert. Der Katalysator wurde abfiltriert, mit Toluol nachgewaschen und das Filtrat eingeengt. Restliches Lösungsmittel wurde mit Hilfe von Toluol azeotrop abdestilliert. Kristallisation aus einem Gemisch von Essigsäureethylester und Methanol (2:1) lieferte 2,7 g des Monoacetats der Titelverbindung, Fp. >217 °C (Zersetzung).

### Beispiel 6:

### 4-{4-[2-(5-Amidinopyridyl)]-1-piperazinyl}-phenoxyessigsäure

2,0 g 4-{4-[2-(5-Amidinopyridyl)]-1-piperazinyl}-phenoxyessigsäure-tert.-butylester-monoacetat (Herstellung siehe Beispiel 5) wurden in 20 ml 6-normaler wäßriger Salzsäure gelöst und 1 Stunde lang bei Raumtemperatur gerührt. Das auskristallisierte Trishydrochlorid der Titelverbindung wurde abfiltriert, mit wenig Wasser gewaschen und anschließend getrocknet. Ausbeute: 1,6 g, Fp. = 300 °C (Zersetzung).

### Beispiel 7:

### 4-{4-[2-(5-Amidinopyridyl)]-1-piperazinyl}-phenoxyessigsäuremethylester

353 mg 4-{4-[2-(5-Amidinopyridyl)]-1-piperazinyl}-phenoxyessigsäure-trishydrochlorid (Herstellung siehe Beispiel 6) wurden mit 26 ml 1-normaler methanolischer Salzsäure aufgenommen und 3 Stunden lang bei Raumtemperatur gerührt. Das ausgefallene Produkt wurde abgesaugt, mit kaltem Methanol gewaschen und getrocknet. Man erhielt 310 mg des Trishydrochlorids der Titelverbindung,
Fp. = 151-154 °C.

### Beispiel 8:

### 4-{4-[2-(5-Amidinopyridyl)]-1-piperazinyl}-phenoxyessigsäureisopropylester

460 mg 4-{4-[2-(5-Amidinopyridyl)]-1-piperazinyl}-phenoxyessigsäure-tert.-butylester-monoacetat (Herstellung siehe Beispiel 5) wurden mit 26 ml einer 2-normalen Lösung von Salzsäure in Isopropanol aufgenommen und 3 Stunden lang bei 90 °C gerührt. Das ausgefallene Produkt wurde abgesaugt, mit kaltem Isopropanol gewaschen und getrocknet. Man erhielt 360 mg des Trishydrochlorids der Titelverbindung, Fp. = 157 - 162 °C.

### Beispiel 9 :

### 3-{4-[4-(2-(5-Amidinopyridyl))-1-piperazinyl]-phenyl}-propionsäure-tert.-butylester

A) 4,3 g des 4-(1-Piperazinyl)-phenylpropionsäure-tert.- butylester (Herstellung siehe Beispiel 4C) wurden zusammen mit 2,08 g 2-Chlorpyridin-5-carbonitril und 3,0 g Kaliumcarbonat in 100 ml N-Methylpyrrolidon gelöst und 5 Stunden lang auf 120 °C erhitzt. Die ausgefallenen Salze wurden abfiltriert und der Ansatz anschließend eingedampft. Der Rückstand wurde mit Methylenchlorid aufgenommen und mit Wasser gewaschen. Nach dem Trocknen wurde eingeengt und aus einem Gemisch aus Isopropanol und Ethanol kristallisiert. Man erhielt 3,85 g 3-{4-[4-(2-(5-Cyanopyridyl))-1-piperazinyl]-phenyl}-propionsäure-tert.-butylester.
B) 3,80 g des unter A) erhaltenen tert.-Butylesters, 4 mal 0,32 g Hydroxylaminhydrochlorid und 4 mal 0,48 g Kaliumtert.-butylat wurden nach der in Beispiel 1C) beschriebenen Methode umgesetzt. Die Ausbeute betrug 1,80 g 3-{4-[4-(2-(5-Hydroxyamidino-pyridyl))-l-piperazinyl]-phenyl}-propionsäure-tert.-butylester nach Umkristallisation aus Methanol/Methylenchlorid.
C) 1,0 g des vorstehend erhaltenen Hydroxyamidins und 0,3 g 10%iger Palladiumkatalysator auf Aktivkohle wurden nach der in Beispiel 1D) beschriebenen Methode umgesetzt. Man erhielt 0,79 g des Acetats der Titelverbindung.
   IR: 2977,1724,1607 cm⁻¹.

### Beispiel 10:

### 3-{4-[4-(2-(5-Amidinopyridyl))-1-piperazinyl]-phenyl}-propionsäure

320 mg 3-(4-[4-(2-(5-Amidinopyridyl))-1-piperazinyl]-phenyl}-propionsäure-tert.-butylester (Herstellung siehe Beispiel 9) wurden in 10 ml 50%iger wäßriger Trifluoressigsäurelösung 20 Stunden lang bei Raumtemperatur gerührt und die Lösung anschließend im Vakuum eingedampft. Das erhaltene Rohprodukt wurde durch Mitteldruckflüssigkeitschromatographie (MPLC) an einem Reversed Phase Kieselgel (RP18) gereinigt. Die Ausbeute an Tetrakistrifluoracetat der Titelverbindung betrug 320 mg,
Fp. = 280 °C (Zersetzung).

Nach den in den vorstehenden Beispielen beschriebenen Verfahren können auch die in der nachfolgenden Tabelle 2 aufgeführten Verbindungen der Formel I hergestellt werden.

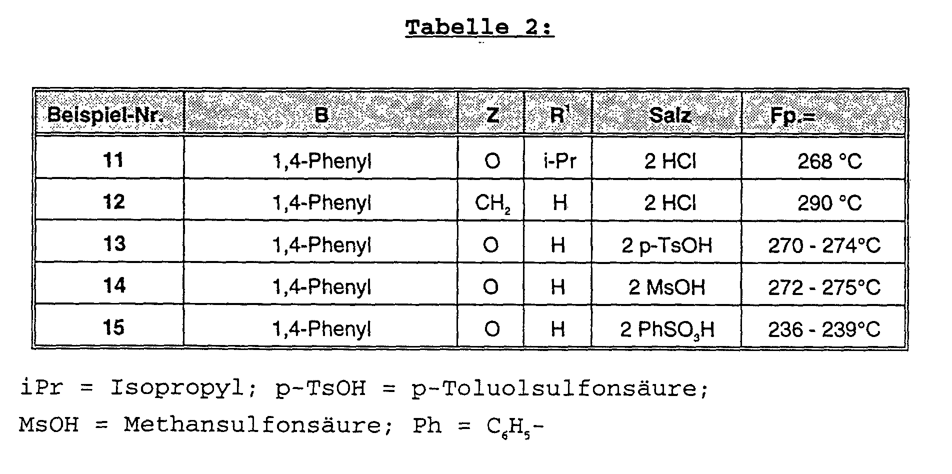

### Beispiel für eine pharmakologische Formulierung

### Beispiel:

### 4-[4-(4-Amidinophenyl)-1-piperazinyl]-phenoxyessigsäuredihydrochlorid enthaltende Tabletten

Man stellte Tabletten in folgender Zusammensetzung pro Tablette her:

| | |
|---|---|
| 4-[4-(4-Amidinophenyl)-1-piperazinyl]-phenoxyessigsäuredihydrochlorid | 20 mg |
| Maisstärke | 60 mg |
| Milchzucker | 135 mg |
| Gelatine (als 10%ige Lösung) | 6 mg |

Der Wirkstoff, die Maisstärke und der Milchzucker wurden mit der 10%igen Gelatine-Lösung eingedickt. Die Paste wurde zerkleinert und das entstehende Granulat wurde auf ein geeignetes Blech gebracht und bei 45 °C getrocknet. Das getrocknete Granulat wurde auf eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Patentansprüche

1. Verbindungen der allgemeinen Formel I 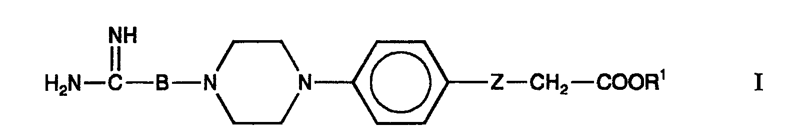 worin
Z Sauerstoff oder eine Methylengruppe bedeutet,
B einen Phenyl- oder Pyridylrest bedeutet, und
R¹ Wasserstoff oder eine einen biolabilen Ester bildende Gruppe bedeutet,
und deren physiologisch verträgliche Säureadditionssalze und physiologisch verträgliche Salze von Säuren der Formel I.

2. Verbindungen gemäß Anspruch 1, worin die einen biolabilen Ester bildende Gruppe R¹ C₁-C₄-Alkyl darstellt.

3. Verbindungen gemäß Anspruch 1, worin R¹ Wasserstoff ist.

4. Verbindungen gemäß einem der vorstehenden Ansprüche, worin Z Sauerstoff bedeutet.

5. Verbindungen gemäß einem der vorstehenden Ansprüche, worin B ein Phenylring ist.

6. 4-[4-(4-Amidinophenyl)-1-piperazinyl]-phenoxyessigsäure und deren C₁-C₄-Ester sowie physiologisch verträgliche Säureadditionssalze dieser Verbindungen und physiologisch verträgliche Salze der freien Säure gemäß Anspruch 5.

7. Arzneimittel, enthaltend eine pharmakologisch wirksame Menge einer Verbindung gemäß Anspruch 1 und übliche pharmazeutische Hilfs- und/oder Trägerstoffe.

8. Verfahren zur Herstellung von Verbindungen der allgemeinen Formel I 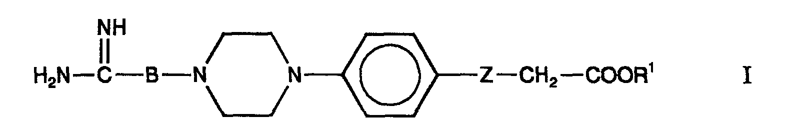 worin
Z Sauerstoff oder eine Methylengruppe bedeutet,
B einen Phenyl- oder Pyridylrest bedeutet, und
R¹ Wasserstoff oder eine einen biolabilen Ester bildende Gruppe bedeutet,
und deren physiologisch verträglichen Säureadditionssalzen und physiologisch verträglichen Salzen von Säuren der Formel I, **dadurch gekennzeichnet, daß** man Verbindungen der allgemeinen Formel IIa 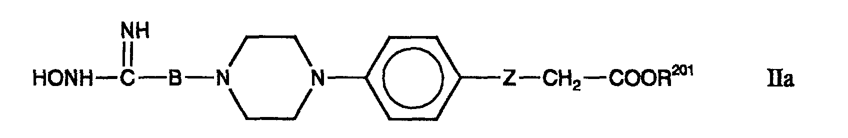 worin Z und B obige Bedeutungen besitzen und R²⁰¹ eine Säureschutzgruppe bedeutet, zu Verbindungen der allgemeinen Formel III 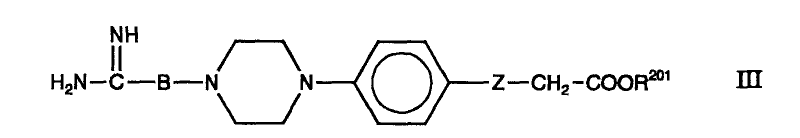 worin Z, B und R²⁰¹ obige Bedeutungen besitzen, hydriert, und, sofern die Säureschutzgruppe R²⁰¹ in den Verbindungen der Formel III nicht eine gewünschte einen biolabilen Ester bildende Gruppe darstellt, diese Säureschutzgruppe zur Freisetzung von Säuren der Formel I abspaltet oder die Verbindungen der Formel III oder daraus erhaltene Säuren der Formel I mit einem Alkohol der allgemeinen Formel IVa
**R**^{**101**}**-OH** IVa
worin R¹⁰¹ die für R¹ angegebene Bedeutung mit Ausnahme von Wasserstoff besitzt, oder dessen reaktiven Derivaten, zu Verbindungen der Formel Ia 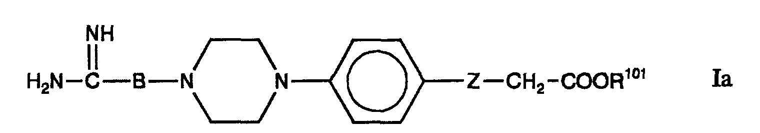 worin Z, B und R¹⁰¹ obige Bedeutungen besitzen, umsetzt, und gewünschtenfalls erhaltene Verbindungen der Formel I in ihre physiologisch verträglichen Säureadditionssalze überführt oder Säuren der Formel I in ihre physiologisch verträglichen Salze überführt oder Säureadditionssalze der Verbindungen der Formel I oder Salze der Säuren der Formel I in die freien Verbindungen überführt.

9. Verbindungen der allgemeinen Formel II 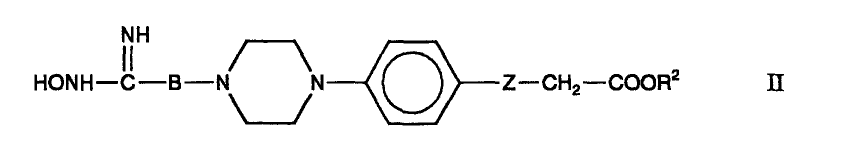 worin
Z Sauerstoff oder eine Methylengruppe bedeutet,
B einen Phenyl- oder Pyridylrest bedeutet, und
R² Wasserstoff oder eine Säureschutzgruppe bedeutet,
und deren Säureadditionssalze und Salze von Säuren der Formel II.

## Claims

1. Compounds of the general formula I 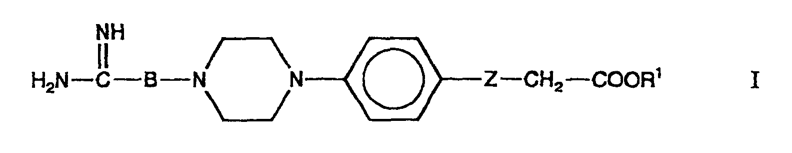 in which
Z is oxygen or a methylene group,
B is a phenyl or pyridyl radical, and
R¹ is hydrogen or a group forming a biolabile ester,
and their physiologically tolerable acid addition salts and physiologically tolerable salts of acids of the formula I.

2. Compounds according to Claim 1, in which the group R¹ forming a biolabile ester is C₁-C₄ alkyl.

3. Compounds according to Claim 1, in which R¹ is hydrogen.

4. Compounds according to one of the above claims, in which Z is oxygen.

5. Compounds according to one of the above claims, in which B is a phenyl ring.

6. 4-[4-(4-Amidinophenyl)-1 -piperazinyl]phenoxyacetic acid and its C₁-C₄ esters, and physiologically tolerable acid addition salts of these compounds and physiologically tolerable salts of the free acid according to Claim 5.

7. Medicaments comprising a pharmacologically active amount of a compound according to Claim 1 and customary pharmaceutical auxiliaries and/or excipients.

8. Process for the preparation of compounds of the general formula I 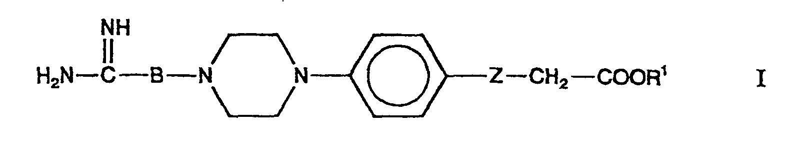 in which
Z is oxygen or a methylene group,
B is a phenyl or pyridyl radical, and
R¹ is hydrogen or a group forming a biolabile ester,
and their physiologically tolerable acid addition salts and physiologically tolerable salts of acids of the formula I, **characterized in that** compounds of the general formula lla 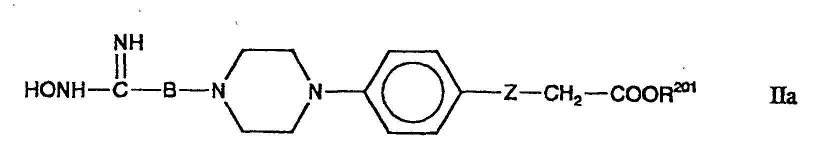 in which Z and B have the above meanings and R²⁰¹ is an acid protective group, are hydrogenated to give compounds of the general formula III 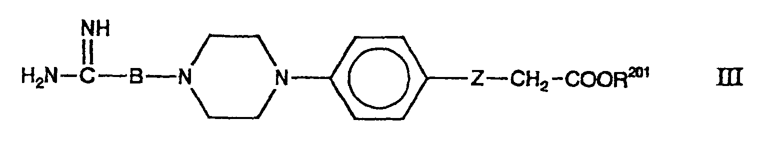 in which Z, B and R²⁰¹ have the above meanings, and, provided the acid protective group R²⁰¹ in the compounds of the formula III is not a desired group forming a biolabile ester, this acid protective group is removed to liberate acids of the formula I, or the compounds of the formula III or acids of the formula I obtained therefrom are reacted with an alcohol of the general formula IVa
R¹⁰¹-OH IVa
in which R¹⁰¹ has the meaning indicated for R¹ with the exception of hydrogen, or its reactive derivatives, to give compounds of the formula la 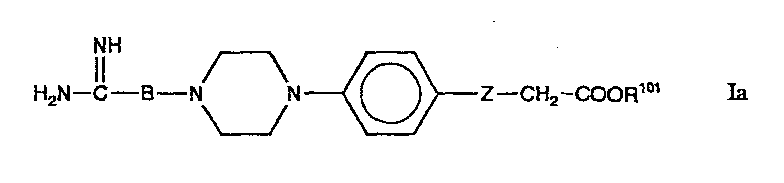 in which Z, B and R¹⁰¹ have the above meanings, and, if desired, compounds of the formula I obtained are converted into their physiologically tolerable acid addition salts or acids of the formula I are converted into their physiologically tolerable salts or acid addition salts of the compounds of the formula I or salts of the acids of the formula I are converted into the free compounds.

9. Compounds of the general formula II 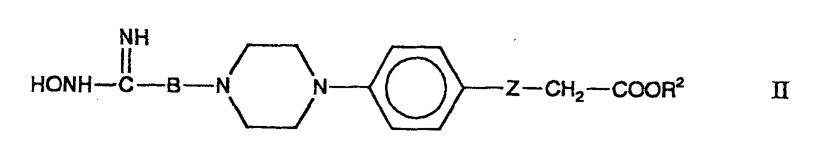 in which
Z is oxygen or a methylene group,
B is a phenyl or pyridyl radical, and
R² is hydrogen or an acid protective group
and their acid addition salts and salts of acids of the formula II.

## Revendications

1. Composés de formule générale I 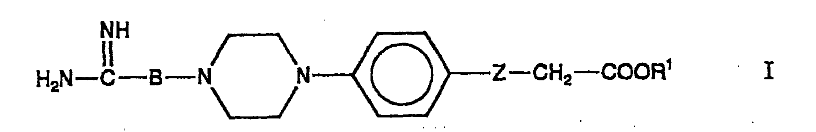 dans laquelle
Z représente un oxygène ou un groupe méthylène,
B représente un radical phényle ou pyridyle, et
R¹ représente un hydrogène ou un groupe formant un ester biolabile,
et leurs sels d'addition d'acides physiologiquement acceptables et les sels physiologiquement acceptables des acides de formule I.

2. Composés selon la revendication 1, dans lesquels le groupe R¹ formant un ester biolabile représente un alkyle en C₁ à C₄.

3. Composés selon la revendication 1, dans lesquels R¹ est un hydrogène.

4. Composés selon l'une des revendications précédentes, dans lesquels Z représente un oxygène.

5. Composés selon l'une des revendications précédentes, dans lesquels B est un noyau phényle.

6. Acide 4-[4-(4-amidinophényl)-1-pipérazinyl]-phénoxyacétique et ses esters en C₁ à C₄ ainsi que les sels d'addition d'acides physiologiquement acceptables de ces composés et les sels physiologiquement acceptables des acides libres selon la revendication 5.

7. Médicament contenant une quantité pharmacologiquement efficace de composé selon la revendication 1, et des adjuvants et/ou supports pharmaceutiques habituels.

8. Procédé de préparation de composés de formule générale I 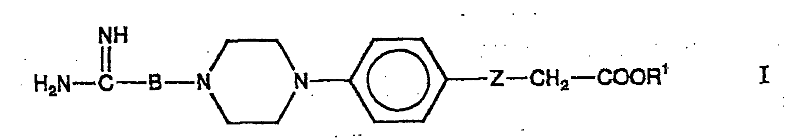 dans laquelle
Z représente un oxygène ou un groupe méthylène,
B représente un radical phényle ou pyridyle,
R¹ représente un hydrogène ou un groupe formant un ester biolabile,
et leurs sels d'addition d'acides physiologiquement acceptables et les sels physiologiquement acceptables des acides de formule I, **caractérisé en ce qu'**on hydrogène des composés de formule générale IIa 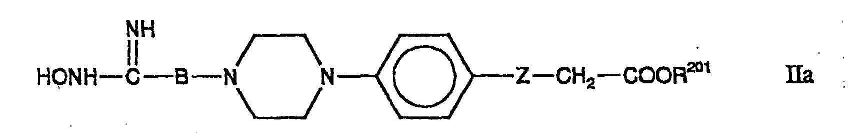 dans laquelle Z et B ont les significations indiquées ci-dessus et R²⁰¹ représente un groupe protecteur d'acide, pour donner des composés de formule générale III 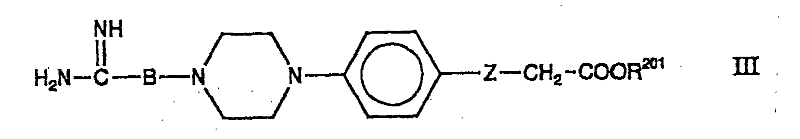 dans laquelle Z, B et R²⁰¹ ont les significations données ci-dessus et, dans la mesure où le groupe protecteur d'acide R²⁰¹ dans les composés de formule III ne représente pas un groupe désiré formant un ester biolabile, on sépare ce groupe protecteur d'acide pour libérer les acides de formule I ou on fait réagir les composés de formule III ou les acides de formule I qu'on en obtient avec un alcool de formule générale IVa
R¹⁰¹―OH IVa
dans laquelle R¹⁰¹ a la signification indiquée pour R¹ à l'exception de l'hydrogène, ou ses dérivés réactifs, pour donner des composés de formule Ia 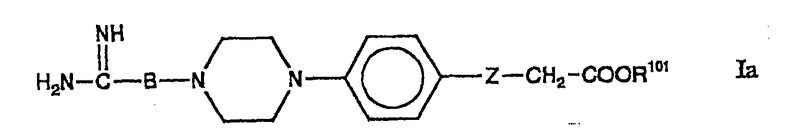 dans laquelle Z, B et R¹⁰¹ ont les significations données ci-dessus, et si on le désire on transforme des composés de formule I éventuellement obtenus en leurs sels d'addition d'acides physiologiquement acceptables, ou on transforme des acides de formule I en leurs sels physiologiquement acceptables, ou on transforme les sels d'addition d'acide des composés de formule I ou les sels des acides de formule I en les composés libres.

9. Composés de formule générale II 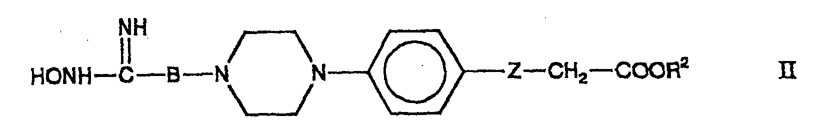 dans laquelle
Z représente un oxygène ou un groupe méthylène,
B représente un radical phényle ou pyridyle, et
R² représente un hydrogène ou un groupe protecteur d'acide,
et leurs sels d'addition d'acides et les sels d'acides de formule II.
